# EUROPEAN PATENT SPECIFICATION

(11) **EP 3 405 785 B1**
(45) Date of publication and mention of the grant of the patent: **29.04.2020**
(21) Application number: 17741166.7
(22) Date of filing: 20.01.2017
(51) Int. Cl.: G01N 33/00, G01N 33/68, G01N 33/74

(54) **BIO-ANALYTICAL METHOD FOR INSULIN ANALOGUES**
BIOANALYSEVERFAHREN FÜR INSULINANALOGA
PROCÉDÉ BIOANALYTIQUE POUR ANALOGUES D'INSULINE

(30) Priority: 23.01.2016 IN 201641002615
(43) Date of publication of application: 28.11.2018
(73) Proprietor: Biocon Limited, Bangalore 560 100 Karnataka (IN)
(72) Inventor: BUDDHA, Madhavan, Karnataka Bangalore 560068 (IN); PATALE, Mukesh, B., Karnataka Bangalore 560081 (IN); KHEDKAR, Anand, Karnataka Bangalore (IN); TAGORE, Ranitendranath, Karnataka Bangalore 560100 (IN); MCDONALD, Sebastian, Alastair, York North Yorkshire YO26 5NJ (GB)
(74) Representative: Michalski Hüttermann & Partner Patentanwälte mbB
(86) International application number: PCT/IB2017/050303
(87) International publication number: WO 2017/125885

(56) References cited:
- KR-A- 20080 067 625
- US-A1- 2014 273 044
- US-B2- 8 058 391
- TAYLOR STEVEN W ET AL: "A high-throughput mass spectrometry assay to simultaneously measure intact insulin and C-peptide", CLINICA CHIMICA ACTA, ELSEVIER BV, AMSTERDAM, NL, vol. 455, 25 January 2016 (2016-01-25), pages 202-208, XP029445214, ISSN: 0009-8981, DOI: 10.1016/J.CCA.2016.01.019
- RODRIGUEZ-CABALEIRO ET AL.: 'Pilot study for the standardization of insulin immunoassays with isotope dilution-liquid chromatography/tandem mass spectrometry' CLINICAL CHEMISTRY vol. 53, no. 8, 2007, pages 1462 - 1469, XP055402970
- KIPPEN ET AL.: 'Development of an isotope dilution assay for precise determination of insulin, C-peptide, and proinsulin levels in non-diabetic and type II diabetic individuals with comparison to immunoassay' JOURNAL OF BIOLOGICAL CHEMISTRY vol. 272, no. 19, 1997, pages 12513 - 12522, XP002272400
- THEVIS ET AL.: 'Qualitative determination of synthetic analogues of insulin in human plasma by immunoaffinity purification and liquid chromatography tandem mass spectrometry for doping control purposes' ANALYTICAL CHEMISTRY vol. 77, no. 11, 2005, pages 3579 - 3585, XP055105086
- STÖCKLIN ET AL.: 'A stable isotope dilution assay for the in vivo determination of insulin levels in humans by mass spectrometry' DIABETES vol. 46, no. 1, 01 January 1997, pages 44 - 50, XP001036780 DOI: 10.2337/DIABETES.46.1.44
- THEVIS ET AL.: 'Doping Control Analysis of Intact Rapid-Acting Insulin Analogues in Human Urine by Liquid Chromatography Tandem Mass Spectrometry' ANALYTICAL CHEMISTRY vol. 78, no. 6, 2006, pages 1897 - 1903, XP055105122

## Description

### FIELD OF INVENTION:

The present invention relates to a specific and sensitive bio-analytical method for detection of insulin or insulin analogues in plasma, serum or any other biological fluid. It particularly relates to labelling of biological compound with stable isotopic nitrogen and bio-analytical method using solid phase extraction and liquid chromatography with tandem mass spectrometric detection.

### BACKGROUND OF INVENTION:

For proteins based biological drugs, it is necessary to detect proteins such as insulin contained in the plasma, serum or any other biological fluid, and measure them quantitatively in order to elucidate the function of the protein, or for testing and screening of the protein, or to measure the systemic exposure of the protein. Therefore, if a more general method for quantifying the amount of insulin with a high sensitivity is established, it becomes possible to estimate drug kinetics during preclinical testing or in clinical trials. Thus, it would be possible to estimate drug kinetics in human at an early stage of drug development.

Typically, radioimmunoassay (RIA) or ELISA methods have been used for quantifying insulin in biological samples. Attempts to generate antibodies specific to Insulin analogue 'IN-105' were not successful, as the immunisation cycles yielded antibodies which showed identical binding to human insulin and IN-105, indicating that the addition of the small alkyl polyethylene glycol (PEG) does not alter the structure significantly enough to differentiate the molecule immunologically. The difference in molecular weight between the two molecules is 217 Daltons due to the alkyl PEG in IN-105. Hence, a specific ELISA based estimation assay could not be developed for IN-105.

Conventionally, a method using electrophoresis such as two-dimensional electrophoresis was conducted for quantifying biogenic proteins. With this method, detection and quantification were performed by staining the protein to be quantified, or by autoradiography, or by using an antibody specific to a particular protein (western blot).

However, it was difficult to apply these methods for quantifying a high molecular protein like insulin or insulin analogue since it is not possible to electrophorese insoluble proteins or high molecular proteins.

On the other hand, there have been significant advances in the field of mass spectrometry, and the technique have been considered and used for detecting or measuring various biological materials. Typically an LC/MS/MS instrumentation involve liquid chromatography (LC) for separation of analytes, followed by ionisation chamber where the sample undergoes desolvation and ionisation and the masses are then detected by mass spectrometer. Advancements in the design of mass spectrometers supported consistent fragmentation of such a large molecule in its intact form, allowing the use of multiple reaction monitoring (MRM) mode, making the method highly specific to IN-105.

The human insulin protein is composed of 51 amino acids, and has a molecular mass of 5808 Da. It is a dimer of an A-chain and a B-chain, which are linked together by disulphide bonds. Several analogues of human insulin are available such as IN-105, Insulin Aspart, Insulin Lispro, and Insulin Glargine. These insulin analogues are closely related to the human insulin structure, and were developed for specific aspects of glycaemic control in terms of fast action (prandial insulin) and long action (basal insulin).

A specific LC/MS/MS method could be developed for measurement of intact IN-105 molecule in plasma. Sample preparations could be carried out using offline solid phase extraction of the analyte, followed by online solid phase extraction before analysis of the samples by LC/MS/MS.

### OBJECT OF INVENTION:

The object of present invention is to provide a sensitive analytical method for the detection and quantification of insulin or insulin analogues IN-105 in human plasma at the concentration range of 0.20 ng/ml to 50.0 ng/ml.

### SUMMARY OF INVENTION:

The present invention relates to a bio-analytical method for the detection of insulin or insulin analogues such as IN-105 in human plasma.

The method comprises labelling of insulin or insulin analogue by a stable isotope of nitrogen i.e. ¹⁵N. Labelling is attained by stable isotope of nitrogen (¹⁵N) by providing labelled nitrogen in growth and fermentation medium. The cells are grown in labelled medium therefore almost all nitrogen atoms in insulin or insulin analogues are substituted with stable isotope ¹⁵N. The labelled nitrogen source used for the method is ammonium sulphate [(¹⁵NH₄)₂SO₄].

Another aspect of the present invention relates to determination of intact molecule of the labelled insulin or insulin analogues which is further determined using solid phase extraction and liquid chromatography with mass spectrometric detection.

### BRIEF DESCRIPTION OF DRAWINGS:

Fig 1 is flowchart of scheme of overall process
Fig 2 is flowchart of upstream process
Fig 3 is process of conversion of IN-105 precursor into IN-105 through trypsin and carboxypeptidase B treatment.
Fig 4 is chromatography peak for Reagent Blank
Fig 5 is chromatography peak for Double Blank
Fig 6 is chromatography peak for Matrix Blank
Fig 7 is Calibration Standard at 0.200 ng/mL LLOQ (Lower Limit of Quantification)
Fig 8 is Calibration Standard 50.0 ng/mL ULOQ (Upper Limit of Quantification)
Fig 9 is QC 0.200 ng/mL LLOQ
Fig 10 is QC 0.600 ng/mL LoQC
Fig 11 is QC 20.0 ng/mL MeQC
Fig 12 is QC 40.0 ng/mL HiQC
Fig 13 is AUCt v DOSE graph of pharmacokinetic study
Fig 14 is Cₘₐₓ v DOSE graph of pharmacokinetic study
Fig 15 is Plot of Mean Plasma Concentration for IN-105 30mg

### DESCRIPTION OF INVENTION:

Present invention relates to bio-analytical method for detection of insulin or insulin analogues using liquid chromatography with tandem mass spectrometry.

In particular, the present invention relates to a highly specific method for quantification of IN-105 in biological matrix.

In one of the embodiments, biological matrix is whole blood, blood serum, blood plasma, urine, fermentation broth or buffer.

The present method allows us to determine IN-105 exposure under variety of disease conditions. Method can distinguish IN-105 from co-administered insulin/insulin analogue and endogenous insulin. Due to the use of mass spectrometry, the method can be used to determine different insulin analogues simultaneously as long as there is a difference in the molecular weight. The method can be further developed to additionally determine C-peptide levels along with insulin or its analogues. The method in general provides a useful alternative to immunological methods.

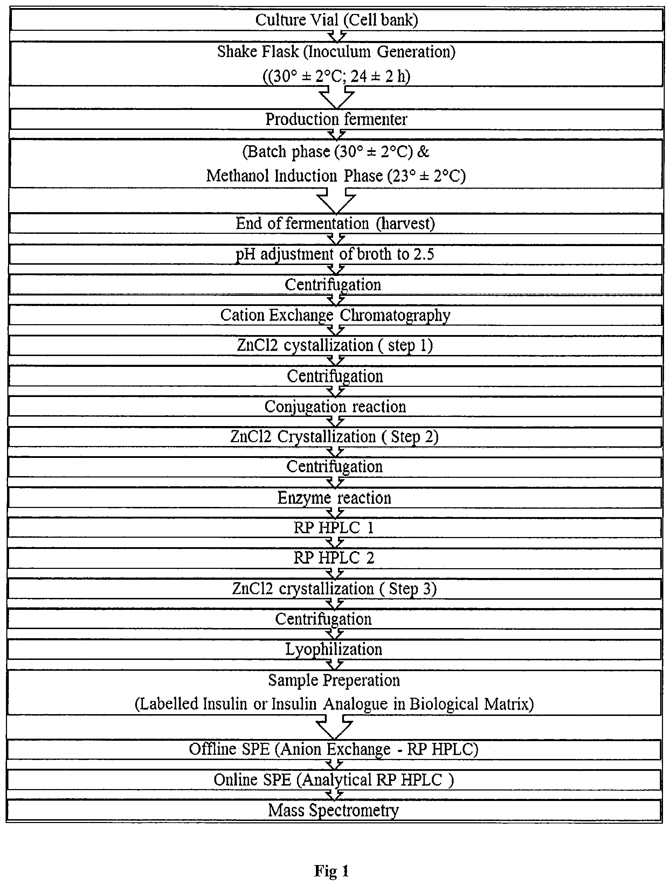

The scheme of the process is depicted in Fig 1.

IN-105, is a novel 'rapid acting insulin analogue' being developed for oral delivery as disclosed and described in US 9,101,596. The molecule has an amino acid sequence identical to that of human insulin, except that it has been conjugated with a short chain alkyl-polyethylene glycol molecule at the ε-amino group of lysine at the 29^{th} position of the B-chain.

Due to the high specificity of the method, IN-105 could be selectively quantified in presence of any human insulin or co-administered insulin like insulin Glargine. This makes the method very useful in determining pharmacokinetic exposure of IN-105 following oral administration as demonstrated from the pharmacokinetic profile obtained from 10 mg dosing in patients with T1D (Type 1 Diabetes).

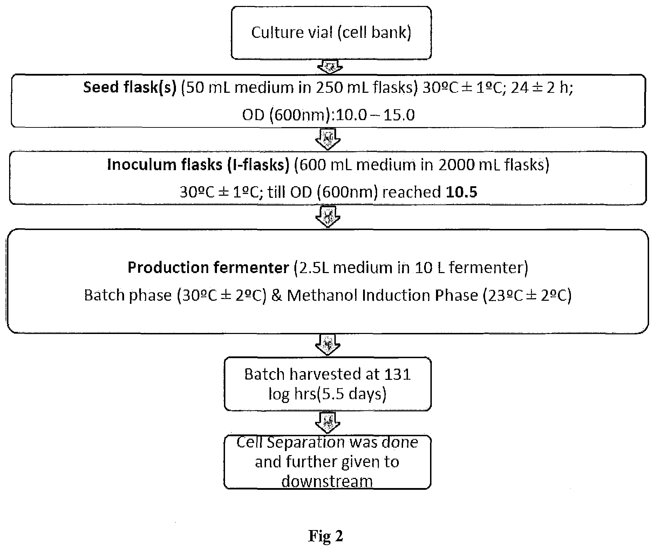

Upstream process involves labelling of insulin or insulin analogues by stable isotopic non-metal elements such as nitrogen (¹⁵N), sulphur (³⁴S), carbon (¹³C), oxygen, or hydrogen. The isotopic nitrogen (¹⁵N) source in the culture medium is at least one of ammonium sulphate, Ammonium phosphate, Ammonium hydroxide, Methylamine, Urea; the isotopic carbon (¹³C) source in the culture medium is at least one of Methanol, Glycerol, Glucose, Sorbitol; isotopic sulphur (³⁴S) source in the culture medium is at least one of Calcium sulphate, Magnesium sulphate, Potassium sulphate; D20 for hydrogen and H₂O¹⁸ for oxygen labelling. The scheme of the process is depicted in Fig 2.

Downstream process involves validation method for determining pharmacokinetic exposure in normal and disease plasma from human subjects. The validation study included linearity, accuracy and precision, selectivity and specificity, repeatability and reproducibility.

The upstream process begins with seed culture preparation, which involves inoculation of seed flasks from the culture of cell bank prepared by using a recombinant strain, *Pichia pastoris.* The strain carries a gene which codes for the expression of insulin precursor. The seed flasks were grown over a period to increase the cell mass which will be used as the inoculum to start the production fermenter. Fermentation is carried out in two phases viz. batch phase and fed batch phase. During batch phase, the fermentation is performed to increase the cell mass using glycerol as carbon source and during fed batch phase methanol is used to induce the secretion of insulin precursor along with ¹⁵N labelled ammonium sulphate as the only nitrogen source during process. The insulin precursor protein with labelled ¹⁵N is released into the supernatant during fermentation.

After fermentation, the downstream purification process serves to isolate the insulin precursor from the cell-free supernatant and convert it into a Methoxy-triethyleneglycol-propionyl-NεB29 recombinant insulin precursor, which through a trypsin and carboxypeptidase B catalysed reaction gets converted into insulin. Multiple HPLC steps are used to resolve close product-related impurities during this process. Multiple chromatography steps are used to resolve product related impurities. After the purification steps, the product is crystallized and lyophilized.

Estimation methods specific to an insulin analogue is useful in clinical investigations in differentiating the exogenously given insulin analogue from endogenous insulin. In the context of IN-105, a method for specifically estimating IN-105 provides unequivocal evidence for absorption of the insulin analogue upon oral delivery. The implications of such a finding are of immense importance in clinical development of oral insulin. IN-105 being a rapid acting insulin, it is expected to be co-administered with a basal insulin e.g. Glargine, under specific therapy conditions. Therefore, specificity of the LC/MS/MS method was tested in presence of basal insulin Glargine.

Upon obtaining ¹⁵N labelled product of insulin or insulin analogue, the product was used as internal standard in analytical method for determination of IN-105 in plasma. Analytical method can be used for the determination of insulin or insulin analogues in human plasma or other biological fluids containing K₂EDTA or K₃EDTA (either Di-potassium or Tri-potassium EDTA).

Analysis employs liquid chromatography - tandem mass spectrometry using TurbolonSpray, in positive ion, multiple reaction monitoring mode. Samples are prepared using off-line Solid-phase extraction (SPE) extraction, followed by on-line SPE in 96-well format.

The method relates to determination of insulin or insulin analogues in human plasma. Preferably, the method is employed to determine insulin or insulin analogues over the concentration range 0.200 ng/mL to 50.0 ng/mL.

### UPSTEAM PROCESS:

Inoculum Flask: The procedure for labelling begins with seeding of cell line in inoculum flask (either directly or through seed flask) that contain medium, designed for same as shown in table 1.

**Table 1: medium composition for inoculum flask**

| | | |
|---|---|---|
| 1 | Composition of Medium: | Yeast nitrogen base (YNB) without amino acid and ammonium sulphate (2.01g) + Ammonium sulphate (6g) |
| | | The above contents were added and volume was made up to 360ml with miliQ water. |
| 2 | Composition of Phosphate buffer: | K₂HPO₄ (2.1g) + KH₂PO₄ (6.5g) |
| | | The above contents were added and volume was made up to 120ml with miliQ water. |
| 3 | Glycerol: | Glycerol (7.5g) |
| | | Glycerol was added and volume was made up to 120ml with miliQ water. |

1. All the three components were separately autoclaved at 121°C for 60min.
2. After autoclaving, the three components were mixed in 2000mL flask and was inoculated with one culture vial.
3. The flask was kept in shaker incubator for incubation at 30°C± 1°C for 24 ± 2 hr.

### Fermentation Procedure

When OD (Optical Density) reached to desired level (10), the inoculum from inoculum flask was transferred further for fermentation. Fermentation involved batch phase and methanol fed-batch.

Composition of fermentation medium described in table 2-4.

**Table 2: Composition of Fermentation Medium**

| **Raw Material** | **Conc. (g/L)** | **Conc. (g/2.5L)** |
|---|---|---|
| Glycerol | 40 | 100.0 |
| H₃PO₄ (Ortho Phosphoric Acid) | 15.7 | 39.3 |
| K₂SO₄ | 18.2 | 45.5 |
| MgSO₄.7H₂O | 14.9 | 37.3 |
| KOH | 2.1 | 5.3 |
| CaSO₄.2H₂O | 0.5 | 1.3 |
| Ammonium sulphate (¹⁵N labeled) | 20 | 50.0 |

**Table 3: Ammonium sulphate feed stock**

| **Age (h)** | **20% (w/v) Ammonium Sulphate (¹⁵N labeled) Feed Rate (g/h)** |
|---|---|
| 22-60 | 4±2 |
| 60-EOF | 12±2 |

**Table 4: Trace Element Composition**

| **Sr. No**. | **Composition** | **Quantity (gm) for 1000ml** |
|---|---|---|
| 1 | Copper Sulphate (CuSO₄.5H₂O) | 6.0 |
| 2 | Manganese Sulphate (MnSO₄.H₂O) | 3.0 |
| 3 | Sodium Iodide (Nal) | 0.08 |
| 4 | Zinc Chloride (ZnCl₂) | 20.0 |
| 5 | Sodium Molybdate (NaMoO₄.2H₂O) | 0.2 |
| 6 | Boric Acid (H₃BO₃) | 0.02 |
| 7 | Cobalt Chloride (CoCl₂) | 0.5 |
| 8 | Iron Sulphate (FeSO₄.7H₂O) | 65.0 |
| 9 | Sulphuric Acid (H₂SO₄) | 5.0 ml |

### Biotin solution preparation:

Biotin (0.2g/L) was dissolved in potable water and filter sterilized through 0.2µ before use.

### Procedure:

1. Fermentation medium was then autoclaved at 121°C for 60min.
2. Fermentation started with 30°C with 350 rpm.
3. After connecting the fermenter, pH was adjusted to 4.9 with 20% w/w NaOH (sodium hydroxide) solution.
4. Sterile trace salt solution and biotin was added (4.35 ml/L of each). As soon as trace salt was added, DO was dropped.
5. 12.0 mL of trace salt solution, 12mL of D-biotin solutions and 2.5gm of 20% H₂SO₄ (w/w) are added per litre of methanol. Methanol is filter sterilized through 0.2µ before feeding into fermenter.
6. 6. Once the OD in the Inoculum flask was reached to 10-15, 250 ml of inoculum was added.
7. 7. RPM was increased from 350 to 800 in 7-8 steps.

### Batch Phase

Parameters of batch phase fermentation are
1. pH: 5.0±0.2
2. Temperature: 30±2°C
3. Dissolved Oxygen (DO): 30%

DO was first maintained by increasing the RPM manually. When DO was reached to 40%, RPM was increased by 200 in one step up to maximum.

Once RPM was reached to maximum, DO (Dissolved Oxygen) was put in cascade of gas mix only starting with minimum of 10% and maximum of 30% and then increased as per the requirement.

When DO started to shoot up and pH started rising, sample was taken and analysis for Wet Cell Weight was done. pH was then changed to set point 6.0 whereas, temperature was adjusted to 23°C.

When batch phase was over, after one hour methanol phase was started.

### Methanol Fed-Batch

Parameters of methanol fed-batch fermentation (Methanol induction phase) are
1. pH: 6.0±0.2
2. Temperature: 23±2°C

Once the DO was raised in batch phase, methanol addition was started. (Refer table 4).

Methanol flow rate was checked periodically by using balance (0.8 density factor). Balance reading were recorded continuously.

The fermentation was checked periodically for methanol accumulation (1-5 min) during methanol induction phase as mentioned in the table 5.

**Table 5: Methanol Induction Phase (MIP) against Methanol Feed Rate.**

| **MIP Age (hrs.)** | **Methanol Feed Rate (g/hrs.)** |
|---|---|
| 0-2 | 2 |
| 3-6 | 3-4 |
| 6-8 | 8-9 |
| 8-10 | 13-15 |
| 10-14 | 17-19 |
| 14-16 | 20-22 |
| 16-Till EOF | 24-25 |

The insulin or insulin analogue precursor protein with Labelled ¹⁵N is released into the supernatant during fermentation.

### Treatment of precursor

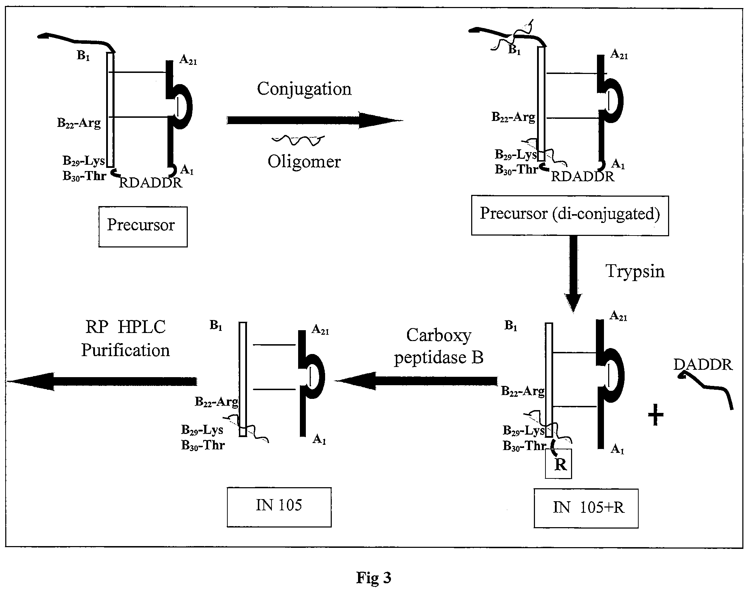

After fermentation, pH of final broth is adjusted to pH 2.5 with ortho-phosphoric acid followed by centrifugation to get cell free supernatant which further subjected to the downstream purification process to isolate the insulin or insulin analogue precursor from the cell-free supernatant and convert it into a Methoxy-triethyleneglycol-propionyl-N-ε-B29 recombinant insulin or insulin analogue precursor, which through a trypsin and carboxypeptidase B catalysed reaction gets converted into insulin or insulin analogue. The scheme of the process of conversion of IN-105 precursor is depicted in Fig 3.

### DOWNSTEAM PROCESS:

Preparation of stock solution, calibration standards and sample

### Control Plasma:

Control Human Plasma (K₂EDTA) from normal healthy volunteers (NHV) was obtained.

Control Human Plasma (K₂EDTA or K₃EDTA) from patients diagnosed with either type one (T1DM) or type two diabetes (T2DM) were also obtained. Plasma from patients with type 1 and type 2 diabetes was procured from external supplier and was available either Di-potassium EDTA (K₂EDTA) or Tri-potassium EDTA (K₃EDTA).

Control Human Blood (K₂EDTA or K₃EDTA) was obtained from volunteers for use in whole blood stability experiment and in preparation of the matrix used in the assessment of haemolysis (2% whole blood in plasma).

Lipaemic plasma was prepared by the addition of intralipid to control human plasma (K₂EDTA) in a ratio 1:9, was used to assess the impact of lipaemia on the method. Haemolysed (2% whole blood in plasma) plasma was also obtained.

All the plasma obtained, either from external supplier or in-house volunteers, was stored at -20 °C prior to use and whole blood was stored at 4 °C.

### Solution preparation:

Two separate stock solutions were prepared by dissolving 2 mg of IN-105 and dissolving in 1 mL of 2% acetic acid containing methanol:water in the ratio of 30:70 v/v. Thus, the stock solution of 2000 µg/mL was prepared.

One was named "calibration stock" and other was named "quality control stock". The stock solution was stored at -20°C for 40 days.

Calibration stock solution was prepared by adding 50 µL of stock solution and diluting it in 450 µL of diluent (0.1% TFA in acetonitrile:water 50:50 v/v).

Quality control (QC) samples were prepared as per table 6 in manner having final concentration of 0.2 (LLOQ QC), 0.6 (LoQC), 20 (MeQC), 40 (HiQC) and 50 (ULOQ QC). These QC samples were stored at -20 °C and -80 °C for a period of 73 days and were used only for validation purpose.

**Table 6: Quality Control Samples**

| Quality Control Samples | | |
|---|---|---|
| Test Samples | | |
| QC | Design | Conc (ng/mL) |
| LLOQ-QC | 6 Replicates | 0.200 |
| LoQC | | 0.600 |
| MeQC | | 20.0 |
| HiQC | | 40.0 |
| ULOQ-QC | | 50.0 |

### Preparation of standards

Various standards prepared for validation study that includes analytical standard, internal standard, co-administered standard and calibration standard.
- Analytical Standard: Standards IN-105 at 96.3% purity were used within its known stability period.
- Internal Standard: Stock solution of internal standard was used in its known stability period and prepared using ¹⁵N labelled IN-105 in the same manner as calibration solutions. The final concentration of internal standard solution was 100 ng/mL at 96.1% purity.
- Co-administered Standard: The co-administered standard 'Glargine' at 3.64mg/mL concentration and 100% purity was used within its known stability period.
- Calibration Standard: The calibration standards were prepared freshly from "calibration stock solution". The calibration standards were prepared using appropriate volume of calibration stock and diluting it with plasma from normal healthy volunteers containing K₂EDTA or K₃EDTA. The calibration standards can be prepared in bulk and stored at -20°C or -80°C for up to 244 days and used within the known stability of IN-105 in human plasma. Table 7 elaborates details of calibration standards used for procedure and its preparation.

The final calibration standard concentrations were 0.2, 0.4, 1, 5, 10, 20, 45 and 50 ng/mL.

**Table 7: Calibration Standard**

| Test Samples | | Preparation | Acceptance criteria based on back calculated concentrations |
|---|---|---|---|
| Concentration (ng/mL) | Replicates | | |
| 0.200 | 2 | Calibration standards | Overall ≥75% of the calibration standard should be within ±15% of nominal (±20% at the LLOQ). |
| 0.400 | 2 | 1. Prepared from calibration stock solution | |
| 1.00 | 2 | | 1. Calibration standards within ±15% of nominal concentration must be included within the calibration line, and |
| 5.00 | 2 | 2. prepared in control human plasma | |
| 10.0 | 2 | 3. prepared freshly for each run | |
| 20.0 | 2 | | 2. Calibration standards with a concentration exceeding ±15% of nominal concentration must be excluded from the calibration line |
| 45.0 | 2 | 4. used within the known stability | |
| 50.0 | 2 | | |

### Sampling

The samples obtained using labelled insulin or insulin analogue in biological matrix by off-line solid phase extraction (SPE) followed by on-line SPE in 96 well formation.

Sampling was done for sterility, wet cell weight (WCW), Titre, pH and conductivity on every day basis (every 24 hrs.).

Quality control samples prepared in quality control working solution in TFA (0.1%) in MeCN:H₂O 50:50, freshly on the day of analysis.

Aliquot (300 µL each) of samples were prepared in a 2 mL 96 well plate. To these aliquots, 25 µL of internal standard (100 ng/mL) was added. None of the blanks were spiked with internal standard. The plate was vortexed for 2 minutes at 1000 rpm. To this, 50 µL of 6 M guanidine HCl was added and the plates were vortexed for 2 minutes at 1000 rpm followed by sonication for 10 minutes. To the wells, 300 µL of 10 mM tris buffer was added and plate was vortexed for 2 min at 750 rpm for mixing.

In one embodiment nitrogen isotope labelled insulin having purity 96.3% and 96.1% respectively, was used as analytical standard and internal standard for further analysis. Glargine (3.64 mg/mL) of 100% purity was used as co-administered standard in the analytical procedure. The stability studies to understand storage stability and solution stability were carried out.

### Stability Study

In one embodiment, for stability assessment of IN-105 in whole blood, IN-105 was spiked in whole blood samples at LoQC and HiQC concentrations. The samples were sub-aliquoted and stored at room temperature and on ice. Aliquots of whole blood were taken up to 2 hrs. The samples were processed for analysis by centrifugation at 4000 rpm at 20°C for 10 minutes. Separated plasma was harvested and stored at -80°C until the time of analysis.

Assessment of sample stability on storage (storage stability) was carried according to table (8), Acceptance criterion was defined as a recovered concentration within ±15% of the nominal spiked concentration. All standards were used within its stability period.

**Table (8): Stability Study**

| | | | |
|---|---|---|---|
| 1 | stability in plasma stored at room temperature | analysed for 24 hr stability | plasma was spiked at LoQC, HiQC, and dilution QC concentrations |
| 2 | stability in plasma exposed to repeated freeze-thaw cycles | assessed for six cycles of freeze-thaw | plasma was spiked at LoQC, HiQC, and dilution QC concentrations |
| 3 | stability in frozen plasma at -20 and -80 °C | assessed for a storage period of 244 days | plasma was spiked at LoQC and HiQC concentrations |

System suitability was tested prior to run. The signal at the LLOQ should be at least 5 times greater than that of the noise and the chromatography and retention time should be consistent with that seen previously. Carry-over greater than 20% of the LLOQ has been observed within the validation and therefore carry-over within the system suitability samples is anticipated but should be no greater than 50% of the LLOQ when directly after the ULOQ and less than 20% of the LLOQ in the blank injection. The samples with be injected such that each type of validation sample, at each concentration level, was distributed throughout the run. Table 9 elaborates system suitability checked prior to run.

**Table 9: elaborates system suitability checked prior to run.**

| **Run description** | **Run contents** |
|---|---|
| Validation of change to analytical method | • Two sets of calibrations standards (front and back) |
| | • Six replicates of each QC |
| | • A reagent blank |
| | • A blank |
| | • A double blank |
| | • Samples to investigate matrix effects (n=12) |
| | • Reference samples for the investigation of matrix effects (n=6) |
| | • Selectivity samples without internal standard (n=6) |
| | • Selectivity samples with internal standard (n=6) |
| | • Duplicate carry-over blanks, run after each ULOQ calibration standard (front and back of the run) |
| | • Sufficient samples of control plasma to make the total run size up to a minimum of 96 samples |

### SPE extraction:

Solid phase extraction was carried out using waters Oasis Max (10 mg) SPE plate. Samples were prepared using off-line SPE extraction followed by on-line SPE. Upon obtaining labelled insulin, calibration standard and quality control standards were prepared.

Prior to each validation run, system suitability samples were analysed. These samples typically consisted of samples at the LLOQ, ULOQ and two blank matrix samples thus allowing qualitative assessment of the quality of the chromatography, sensitivity and carry over; with the exception of analytical runs 1 to 7 where only one matrix blank sample was injected after the ULOQ sample (as mentioned in table 7). Although this was a deviation from the Analytical Method, any carry-over observed was not considered to have impacted on the analytical runs.

### SPE conditions:

The SPE cartridges were conditioned with methanol (300 µL) and equilibrated with 300 µL of water. Samples were loaded on to the plate, for ensuring adequate mixing they were aspirated and dispensed. Plate was washed with 500 µL 1M ammonium acetate in MeCN:water (1:10:90 v/v/v), followed by washing with 500 µL 1M ammonium acetate in MeCN:water (1:50:50 v/v/v). Packing material was dried using maximum pressure. 0.1% Triton X-100 was added to a fresh 1 mL well plate. Samples were eluated with 250 µL of 2% formic acid in MeCN:water (50:50 v/v) to the Triton X-100 containing plate. To each of these eluates 300 µL of 0.1% TFA in water was added and mixed with aspiration and dispensing. Samples were capped and taken for analysis.

### LC/MS/MS conditions:

### Chromatographic conditions:

Mobile phase A was TFA 0.02% in acetonitrile: water (10:90 v/v); Mobile phase B was TFA 0.02% in acetonitrile:water (90:10 v/v). Initial concentration of mobile phase A was 85% till 30s, from 30s to 4 min 30s, mobile phase A changed from 85% to 60% in a linear fashion, over next 10s mobile phase A changed to 10% and stayed at 10% till 5 min 30s. For the equilibration, over next 10s mobile phase A changed back to 85%. The run time was completed at 6 min 30s. Temperature of elution was maintained at 50 °C. Elution was carried out using 0.5 mL/min flow rate. Injection volume was 500 µL. Samples were maintained at 10 °C during the sequence. Wash solvent 1 was 0.01% TFA in acetonitrile:water (10:90 v/v), Wash solvent 2 was 0.01% TFA in methanol:water: Triton-XlOO (70:30:0.05 v/v/v), wash solvent 3 was Ammonia (0.5% of 28%) in methanol:water (80:20 v/v).

### Online extraction conditions:

Cartridges were conditioned using 500 µL methanol at 5 mL/min flow rate. Online cartridges were equilibrated with 500 µL of (0.1% TFA) in MeCN: water (10:90 v/v). Backflush wash was given using 1 mL of (0.1% TFA) in MeCN: water (10:90 v/v) at 2 ml/min flow rate. Following that cartridges were washed using 1 mL of (0.1% TFA) in MeCN: water (20:80 v/v) at 5 ml/min flow rate. Clamp flush 1 was carried out using 500 µL of (0.1% TFA) in MeCN: water (10:90 v/v) at 5 mL/min flow rate, clamp flush 2 was carried out using (0.1% TFA) in MeCN: water (90:10 v/v) at 5 ml/min flow rate and Clamp flush 3 was carried out using 500 µL of (0.1% TFA) in MeCN: water (10:90 v/v) at 5 ml/min flow rate.

### MRM transition:

For IN-105, 1506.9 m/z a four charge state was isolated as parent ion and its transition to 1820 m/z daughter ion was monitored. For ¹⁵N labelled IN-105, 1520 m/z was isolated and its transition to 1838.5 m/z daughter ion was monitored for quantification. The molecular weights and charge states of human insulin along with transitions monitored in the MRM mode and summarized as follows in table 10.

**Table (10): molecular weights and charge states of human insulin along with transitions monitored in the MRM mode**

| Species type | Average Molecular weight (Da) | (M + 4H)⁺⁴ (Da) | (M + 5H)⁺⁵ (Da) | Ion Transition monitored (Da) |
|---|---|---|---|---|
| Human insulin | 5808 | 1453 | 1162.6 | Not Monitored |
| IN-105 | 6025 | 1507.25 | 1206 | 1507.25 > 1822 |
| [¹⁵N] IN-105 | 6076 | 1520 | 1216 | 1520 > 1838.5 |

### Validation

The validation study included linearity, accuracy and precision, selectivity and specificity, repeatability and reproducibility.

Validation consisted of intra run precision and bias, inter-run precision and bias, dilution integrity, matrix related modification of ionisation, auto-injector carry over, stability in whole blood at room temperature and on ice, freeze thaw stability of plasma over 5 cycles, 24 h room temperature stability of plasma, frozen stability in plasma at -20 and - 80 °C for 73 days, recovery and extract stability at 10 °C.

Additional validation runs were analysed to carry out additional experiments that would not fit within the runs used to assess precision and bias. The runs to assess precision and bias were designed to be as large as a potential study sample run (96 samples). Where additional samples were required to make a validation run as large as a study sample run, then control human plasma samples were used for this purpose.

### Validation Study Design

Following table 11 elaborate parameters of validation study.

**Table 11: Summarized validation study * analysed after 20-fold dilution with control plasma. ^{Δ}Investigated in plasma from normal healthy volunteers (NHV) & patients with T1DM or T2DM**

| **Parameter investigated** | **Nominal Insulin concentrations** | | | | | | | **Number of replicates** |
|---|---|---|---|---|---|---|---|---|
| | **0** | **0.200** | **0.600** | **20.0** | **40.0** | **50.0** | **500*** | |
| Intra-run precision and bias (investigated in three runs) | | X | X | X | X | X | | 6 |
| Inter-run precision and bias (investigated in three runs) | | X | X | X | X | X | | 18 |
| Dilution integrity | | | | | | | X | 6 |
| Matrix related modification of ionisation^{Δ} | | | X | | X | | | 6 |
| Auto injector carry-over (investigated in all runs) | X | | | | | | | 4 |
| Stability in whole blood at room temperature and on ice | | | X | | X | | | 3 |
| Freeze/thaw stability in plasma (5 cycles) | | | X | | X | | X | 6 |
| Room temperature stability in plasma (24 hours) | | | X | | X | | X | 6 |
| Frozen stability in plasma^{Δ} (-20°C and -80°C for 73 days) | | | X | | X | | | 3 |
| Recovery | | | X | X | X | | | 3 |
| Extract stability at 10°C | | X | X | X | X | X | | 6 |

In the first analytical run, a calibration standard which was prepared at a concentration of 40.0 ng/mL and not at 45.0 ng/mL as per the study plan. All other runs contained calibration standards at the concentrations specified within the study plan. The mean intra-run precision was found to be less than, or equal to 11.1% and the mean intra-run bias was between 0.4% and 3.0%. The inter-run precision and bias was 16.5% and 2.5% at the LLOQ QC concentration, at all other levels the inter-run precision was equal to or less than 8.9% and the inter-run bias was between 0.3% and 2.0%.

In addition to the experiments described above, selectivity of the method in plasma (K₂EDTA/ K₃EDTA) from different sources, effect of lipaemia and haemolysis, selectivity and modification of ionisation of insulin in the presence of Glargine and stability of solutions of insulin or insulin analogues investigated.

Thorough check was performed for stability of plasma at various conditions.

For a calibration curve to be acceptable, 12 (75%) of the calibration standards had to have a back-calculated concentration of IN-105 within ±15% of the nominal value (±20% for the LLOQ). For a given calibration curve, calibration standards had to be included in the regression if the back-calculated concentration deviated from the nominal by less than, or equal to, 15% (20% at the LLOQ). A calibration standard had to be omitted from the regression if the calculated concentration of IN-105 deviated by more than 15% from the nominal (20% at the LLOQ). Up to four calibration standards (25%) could be omitted from the calibration curve, to achieve the acceptance criteria; otherwise the run was rejected. The acceptance criteria for mean intra-run and the inter-run precision was less than, or equal to, 15% at all levels, other than at the LLOQ, at which precision had to be better than, or equal to, 20%. The mean intra-run and inter-run bias had to be within ±15% of the nominal concentration at all levels, other than at the LLOQ, at which bias had to be within ±20% of the nominal concentration.

The study design for these additional validation experiments are detailed in Table 12 below:

**Table 12: Additional validation procedure Investigated in plasma from normal healthy volunteers (NHV)**

| **Parameter investigated** | **Nominal Insulin concentrations** | | | | | | **Number of replicates** |
|---|---|---|---|---|---|---|---|
| | **0** | **0.200** | **0.600** | **20.0** | **40.0** | **50.0** | |
| Intra-run precision and bias (investigated in one run) | | X | X | X | X | X | 6 |
| Matrix related modification of ionisation^{Δ} | | | X | | X | | 6 |
| Selectivity (with and without internal standard) | X | | | | | | 6 |
| Auto injector carry-over (investigated in all additional runs) | X | | | | | | 4 |
| Frozen stability in plasma* (-20°C and -80°C for 244 days) | | | X | | X | | 3 |
| Extract stability at 10°C | | X | X | X | X | X | 6 |

| | | | | | | | |
|---|---|---|---|---|---|---|---|
| *Though not part of the study design to determine the validity of the modification made to the Analytical Method was investigated using the modified Analytical Method | | | | | | | |

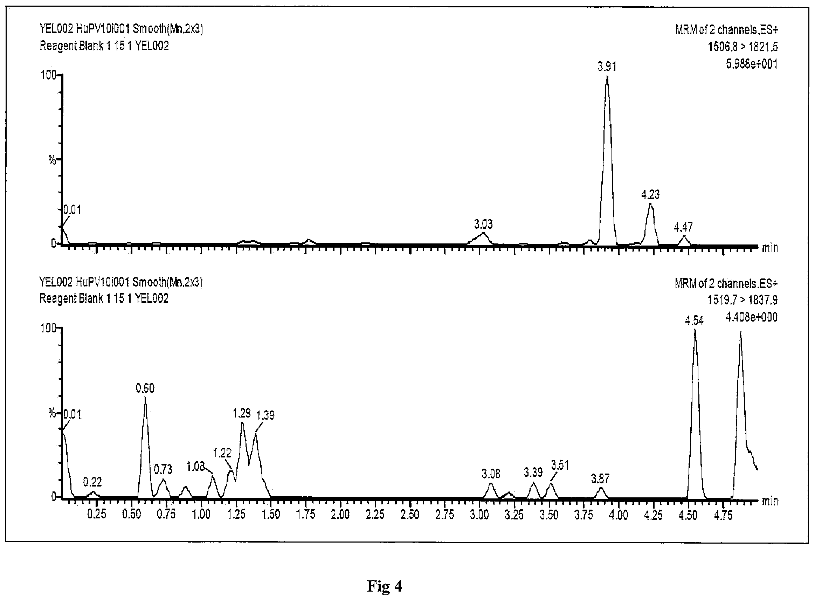

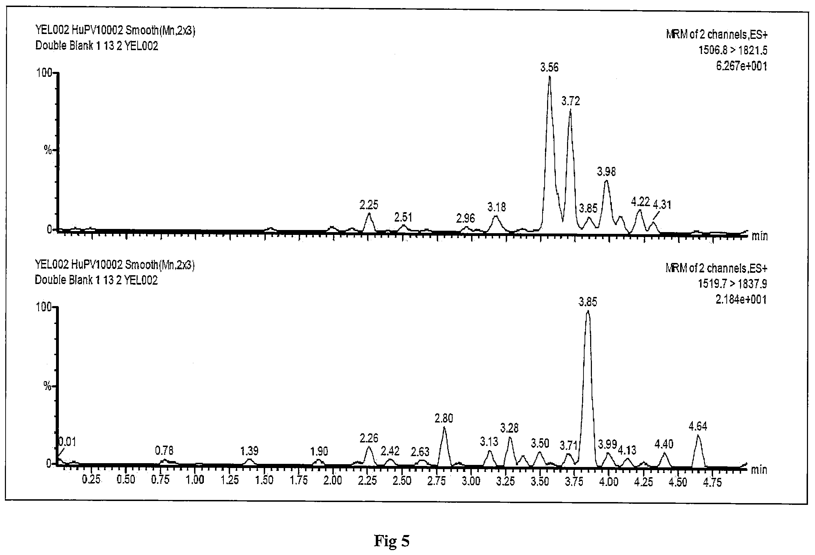

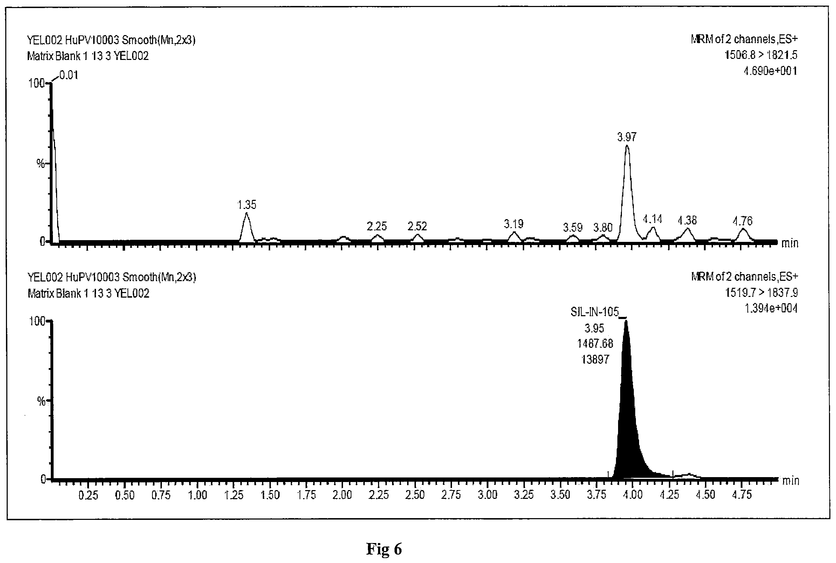

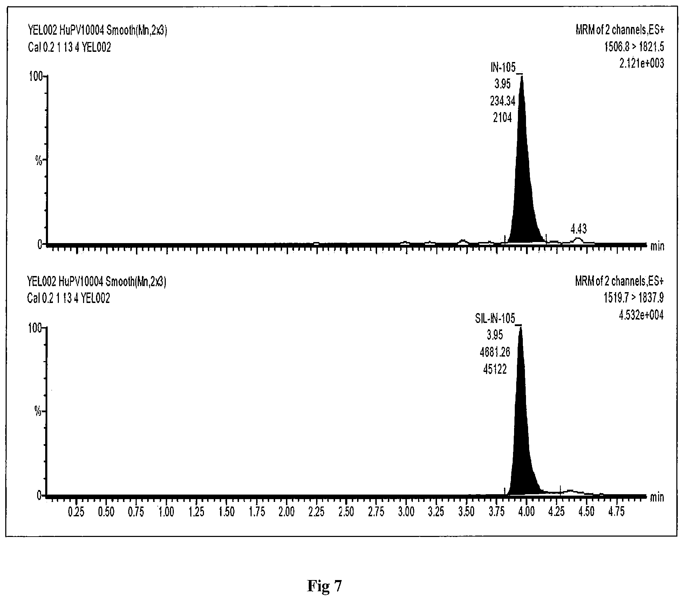

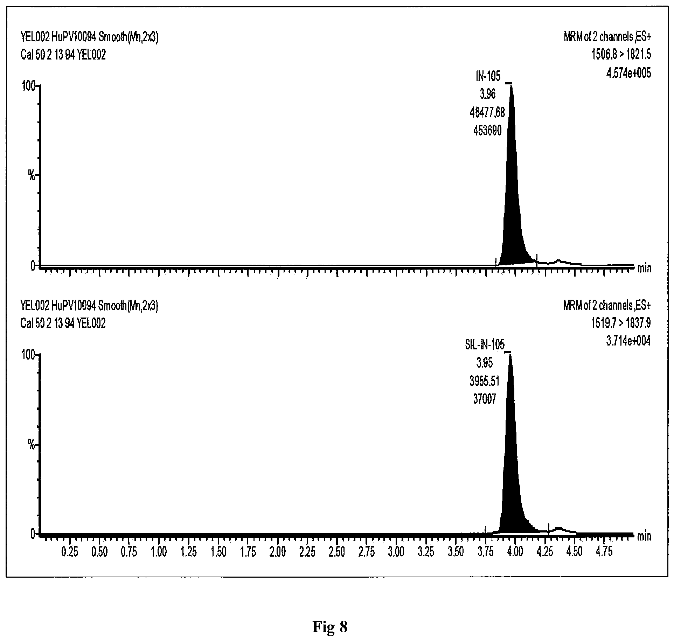

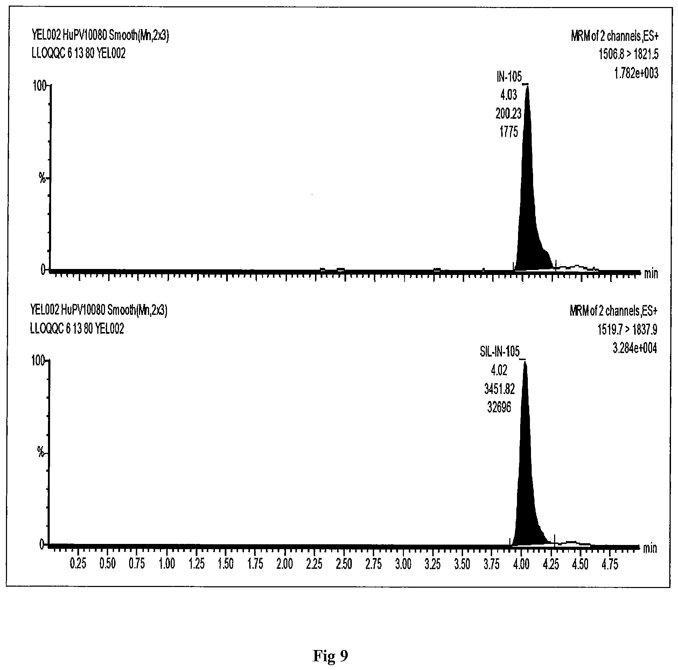

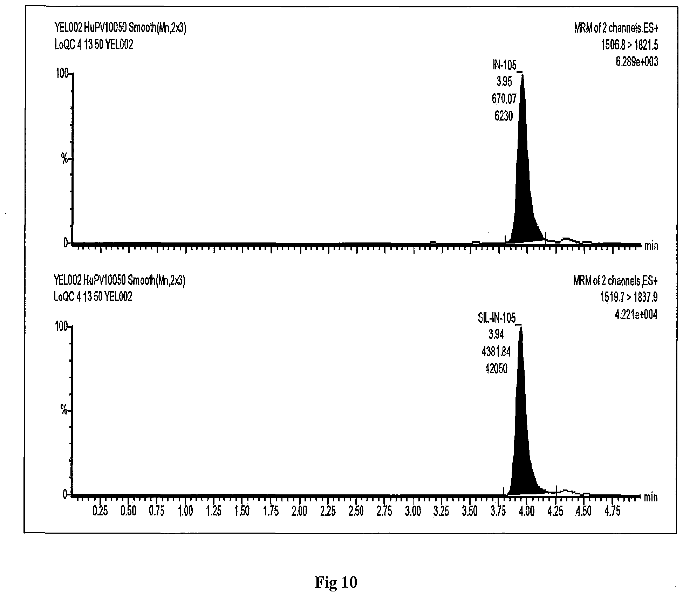

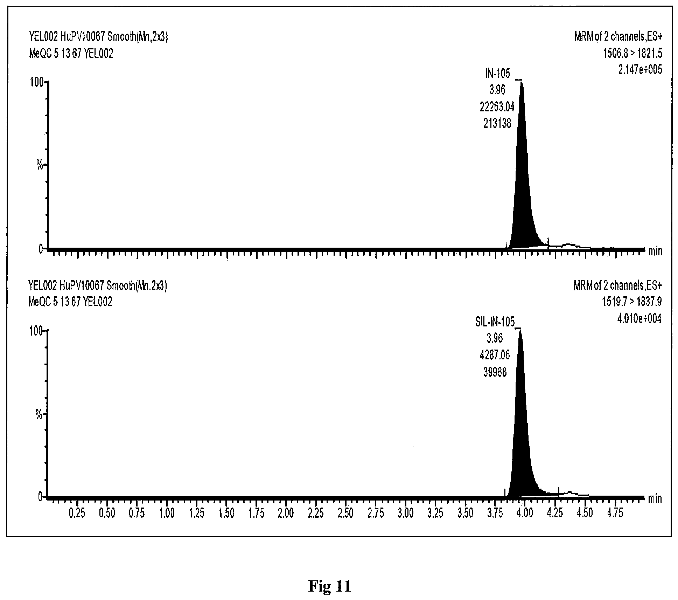

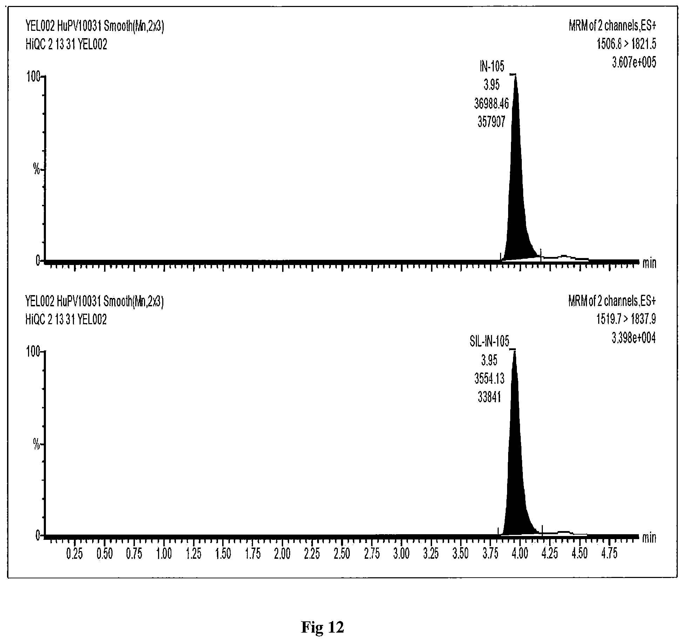

Fig 4-12 show the peaks obtained during LC/MS/MS of samples.

The method was not affected by inter-individual variability, lipaemia, and haemolysis or by presence of Glargine. The observation infers that method is sufficiently accurate and precise and have sufficient selectivity and reliability that allow determination of insulin in human plasma samples over the examined range.

Insulin was found to be stable in plasma when stored at room temperature for up to 24 hours, after five freeze/thaw cycles and after 244 days storage at both -20°C and -80°C. Also found to be stable in extracts when stored at 10°C for approximately 85 hours.

Labelled insulin was stable in acetic acid (2%) in methanol: water (30:70 v/v) when stored at -20°C for up to 40 days and when stored at room temperature for up to 24 hours.

### Example 1: The detection of isotope nitrogen labelled IN-105

Blood samples were collected in tubes containing K2-EDTA. 15N-labelled IN-105 was used as an internal reference standard (ISTD) and added to tubes after separation of serum or plasma. The ISTD-spiked samples were subjected to a mixed-mode anion exchange-reverse phase solid phase extraction process in a 96-well format. The eluate from the mixed-mode SPE are transferred to a reverse-phase (C8) SPE set up in a 96-well format. This RP SPE was setup online with an analytical reverse phase HPLC system. The eluate from the RP SPE were transferred online to the C18 analytical chromatography column and subjected to reverse phase HPLC.

The eluate from the analytical chromatography column passes into a triple quadrupole mass spectrometer, where an ESI process generates gas-phase ions from the eluate. These gas phase ions were then analysed in MRM mode as follows.

The ions pass into the first quadrupole, where ions with m/z values in a narrow range are selected. Ions within this narrow m/z range are allowed to pass through to the second quadrupole.

At the second quadrupole, intact IN-105 and ISTD molecules (and any other ions with m/z values in the same range) are subjected to a collision-induced fragmentation process in presence of an inert gas. This generates fragment ions characteristic of the parent species that were allowed to pass into the second quadrupole. These fragmented species then pass to the third quadrupole.

In the MRM mode, the third quadrupole will be set to select for ions having m/z values characteristic of fragments generated from IN-105 and ISTD. Fragments from other species that happened to have the same intact m/z as IN-105 and ISTD will be eliminated at this stage.

Only the fragments allowed through the third quadrupole are then detected as an ion current. The peak in the extracted Ion Chromatogram (XIC) for IN-105 and ISTD were both found to be integrated. The ratio of the integrated area of IN-105 is to ISTD was used as a measure of the quantity of IN-105 present in the sample.

### Example 2: Pharmacokinetics Study

The method was considered validated successfully since it passed all the pre-determined criteria in the validation protocol. The validated method was subsequently used for measurement of IN-105 levels in a euglycemic clamp study carried out in patients with Type 1 diabetes. The method for the determination of IN-105 in human plasma has been validated successfully over the concentration range 0.200 ng/ml to 50.0 ng/mL.

Plasma concentration data for each patient and treatment was analysed by a non-compartmental method. The area under plasma level curve for AUC0-t was calculated by the trapezoidal rule. The primary pharmacokinetic parameters (mean ± SD) were Cₘₐₓ, AUClast, Tₘₐₓ and PD parameters were Tₘᵢₙ, Cₘᵢₙ, AUClast. Ratios and 90% CIs of geometric means were calculated for PK and PD parameters from mixed effects model with fixed effects for sequence, period and treatment, and patients within sequence as a random effect for log transformed Cₘₐₓ and AUC. The maximal plasma concentration of IN-105 as well as the area under the PK curve are linearly correlated significantly (p < 0.05) with the dose employed (as per table 16 below).

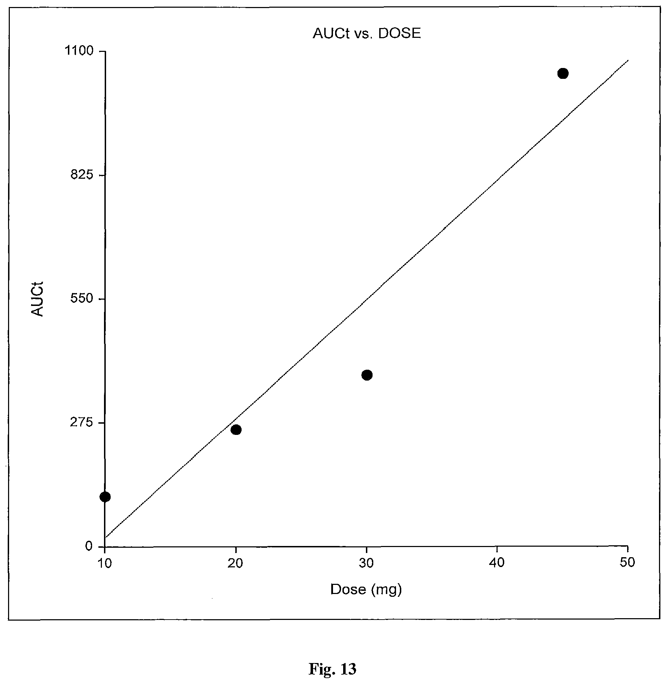

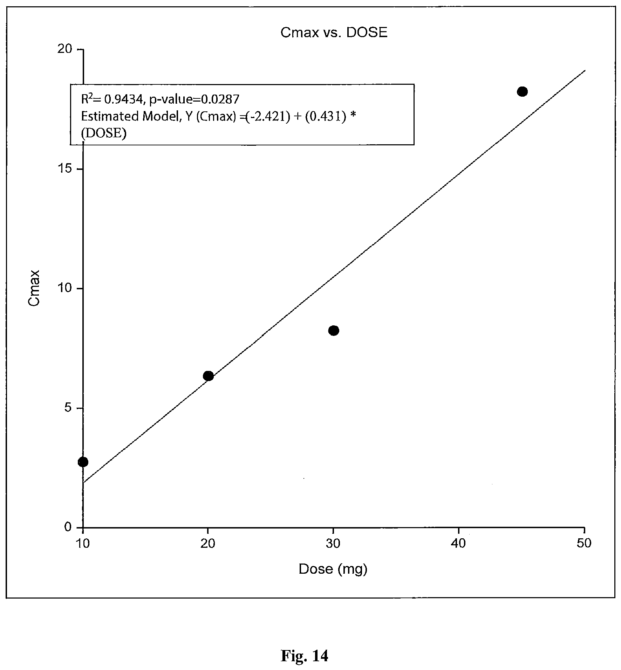

The average plasma concentration time profile of IN-105 obtained after administration of IN-105 tablets in volunteers is shown in figures 13-14.

**Table 16 pharmacokinetic parameters with slope points and p-value**

| **Function of dose** | **slope*** | **Intercept**** | **Regression coefficient (R2)** | **p-value** |
|---|---|---|---|---|
| AUClast (AUCt)(ng.h/ml) | 26.427 | -243.525 | 0.907 | 0.0476 |
| Cₘₐₓ (ng/ml) | 0.431 | -2.421 | 0.9434 | 0.0287 |

| | | | | |
|---|---|---|---|---|
| *area units / mg for AUClast and concentration units/mg for Cₘₐₓ **area units for AUClast and concentration units for Cₘₐₓ | | | | |

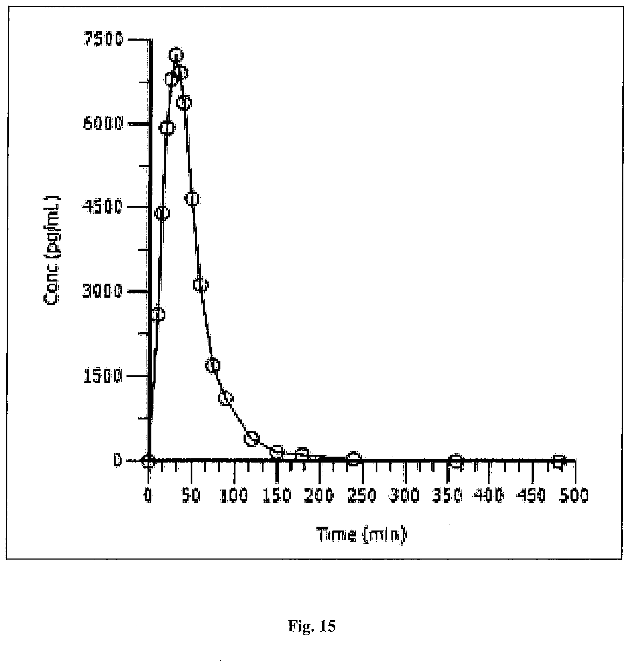

Plots of mean plasma concentration as a function of time after dosing shows the expected time profile of drug concentration in plasma. The plot for the 30 mg/kg dose is shown in figure 15, where a well-defined peak concentration and subsequent clearance is evident.

## Claims

1. A method for selectively detecting and quantifying IN-105 in a biological matrix in the presence of any human insulin or co-administered insulin, including insulin Glargine, wherein the biological matrix is whole blood, blood serum, blood plasma, urine, fermentation broth or buffer, wherein the method comprising the steps of:
i. labelling a known amount of IN-105 by a stable isotope to form a labelled IN-105;
ii. introducing the labelled IN-105 to the biological matrix; and
iii. analysing the biological matrix for intact IN-105 by liquid phase chromatography-tandem mass spectrometry (LC-MS/MS),
wherein IN-105 has the following structure:

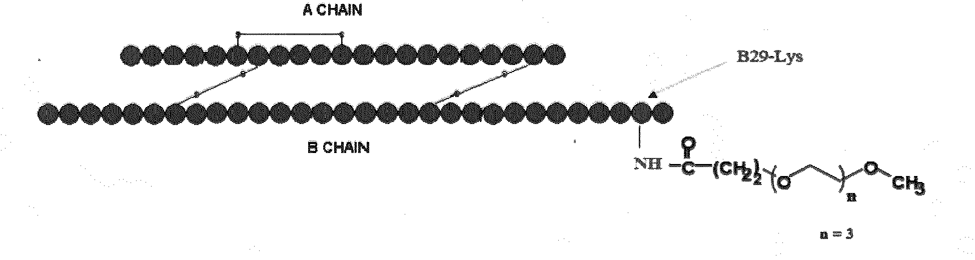

2. The method of claim 1, wherein labelling a known amount of IN-105 by a stable isotope to form labelled IN-105 comprises using a culture medium comprising a recombinant strain of *Pichia pastoris* carrying a gene which codes for expression of pro- IN-105 during a fermentation process.

3. The method of claim 1 or 2, wherein isotope is at least one amongst nitrogen (¹⁵N), sulphur (³¹S), oxygen (¹⁸O), hydrogen (²H) and carbon (¹³C).

4. The method of claim 3, wherein the isotopic nitrogen (¹⁵N) source in the culture medium is at least one of ammonium sulphate, ammonium phosphate, ammonium hydroxide, methylamine, urea, or
wherein the isotopic carbon (¹³C) source in the culture medium is at least one of methanol, glycerol, glucose, sorbitol, or
wherein the isotopic sulphur (³⁴S) source in the culture medium is at least one of calcium sulphate, magnesium sulphate, potassium sulphate.

5. The method of claim 2, wherein the fermentation process is a two-phase process, wherein the first phase is a batch phase to increase cell mass with the inclusion of glycerol as a carbon source and a fed batch phase using methanol to induce secretion of a precursor of the labelled IN-105 and isotope source for inclusion into the expressed labelled IN-105.

6. The method of claim 5, wherein the precursor of labelled IN-105 is treated with trypsin and carboxypeptidase B to form the labelled IN-105.

7. The method of claim 1, wherein the biological matrix is combined with di-potassium or tri-potassium EDTA.

8. The method of claim 1, wherein the biological matrix is stored at -20 °C or at -80 °C or freshly prepared for every cycle.

9. The method of claim 10, wherein the storage period is from 1 day to 250 days.

10. The method of claim 1, wherein step ii) further comprises sample processing using off-line solid-phase extraction (SPE) followed by on-line SPE in 96-well format.

11. The method of claim 1, wherein the labelled IN-105 is used to distinguish exogenously delivered IN-105 from endogenous insulin.

12. The method of any of the preceding claims, being employed to determine IN-105 over a concentration range of 0.200 ng/mL to 50.0 ng/mL.

13. The method of claim 1, comprising:
i) labelling a known amount of IN-105 by the stable isotope of nitrogen to form the labelled IN-105;
ii) introducing the labelled IN-105 to the biological matrix, wherein the biological matrix comprises at least one additional insulin or insulin analogue;
iii) subjecting the solution obtained from step ii) to a mixed-mode anion exchange-reverse phase solid phase extraction process using off-line solid-phase extraction (SPE) followed by on-line SPE in 96-well format; and
iv) analysing the intact labelled IN-105 relative to that of the at least one additional type of insulin or insulin analogue by liquid phase chromatography-tandem mass spectrometry (LC-MS/MS).

14. The use of IN-105, labelled with a stable isotope of nitrogen, for determining the stability of IN-105 in a biological matrix, wherein the biological matrix comprises at least one additional type of insulin or an insulin analogue, the use comprising:
i) labelling a known amount of IN-105 by a stable isotope of nitrogen to form a labelled IN-105,
ii) introducing the labelled IN-105 to the biological matrix,
iii) subjecting the solution obtained from step ii) to a mixed-mode anion exchange-reverse phase solid phase extraction process using off-line solid-phase extraction followed by on-line solid-phase extraction in 96 well-format, and
iv) analysing the intact labelled IN-105 relative to that of the at least one additional type of insulin or insulin analogue by LC-MS/MS.

15. The use of claim 14, wherein the stable isotope of nitrogen is ¹⁵N.

## Patentansprüche

1. Verfahren zum selektiven Nachweisen und zum Quantifizieren von IN-105 in einer biologischen Matrix in Gegenwart eines jedweden Humaninsulins oder gleichzeitig verabreichtem Insulins, einschließlich Insulin Glargin, wobei die biologische Matrix Vollblut, Blutserum, Blutplasma, Urin, Fermentationsansatz oder Puffer, wobei das Verfahren die Schritte aufweist:
i. eine bekannte Menge von IN-105 mit einem stabilen Isotop markieren, um ein markiertes IN-105 zu bilden,
ii. das markierte IN-105 in die biologische Matrix einführen und
iii. die biologische Matrix durch Flüssigphasenchromatographie-Tandem-Massenspektrometrie (LC-MS/MS) auf intaktes IN-105 analysieren,
wobei IN-105 die folgende Struktur hat:

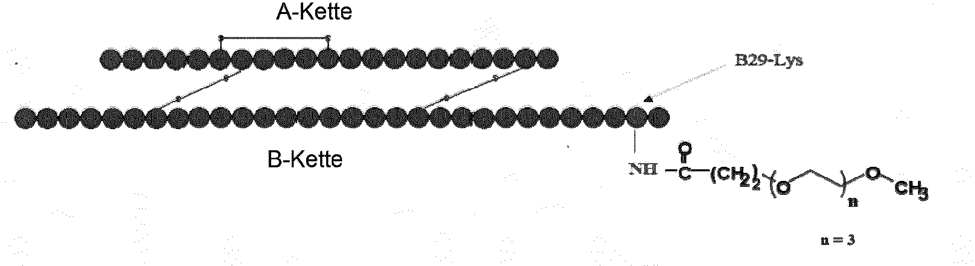

2. Verfahren nach Anspruch 1, wobei eine bekannte Menge von IN-105 mit einem stabilen Isotop markieren, um ein markiertes IN-105 zu bilden, aufweist, ein Kulturmedium zu verwenden, das einen rekombinanten Stamm von *Pichia pastoris* 1668 aufweist, der ein Gen trägt, das während eines Fermentationsprozesses für die Expression von Pro-IN-105 kodiert.

3. Verfahren nach Anspruch 1 oder 2, wobei das Isotop wenigstens eines aus Stickstoff (¹⁵N), Schwefel (³⁴S), Sauerstoff (¹⁸O), Wasserstoff (²H) und Kohlenstoff (¹³C) ist.

4. Verfahren nach Anspruch 3, wobei die Quelle des Isotopenstickstoffs (¹⁵N) in dem Kulturmedium wenigstens eine aus Ammoniumsulfat, Ammoniumphosphat, Ammoniumhydroxid, Methylamin, Harnstoff ist oder
wobei die Quelle des Isotopenkohlenstoffs (¹³C) im Kulturmedium wenigstens eine aus Methanol, Glycerin, Glucose, Sorbit ist oder
wobei die Quelle des Isotopenschwefels (³⁴S) im Kulturmedium wenigstens eine aus Calciumsulfat, Magnesiumsulfat, Kaliumsulfat ist.

5. Verfahren nach Anspruch 2, wobei das Fermentationsverfahren ein Zweiphasenverfahren ist, wobei die erste Phase eine Batch-Phase zum Erhöhen der Zellmasse unter Einbeziehen von Glycerin als Kohlenstoffquelle und eine Fed-Batch-Phase unter Verwendung von Methanol zum Induzieren der Sekretion eines Vorläufer des markierten IN-105 und einer Isotopenquelle zur Aufnahme in das exprimierte markierte IN-105 ist.

6. Verfahren nach Anspruch 5, wobei der Vorläufer des markierten IN-105 mit Trypsin und Carboxypeptidase B behandelt wird, um das markierte IN-105 zu bilden.

7. Verfahren nach Anspruch 1, wobei die biologische Matrix mit Di-Kalium- oder Trikalium-EDTA kombiniert wird.

8. Verfahren nach Anspruch 1, wobei die biologische Matrix bei -20 °C oder bei -80 °C gelagert oder für jeden Zyklus frisch hergestellt wird.

9. Verfahren nach Anspruch 10, wobei die Lagerdauer von 1 Tag bis 250 Tage beträgt.

10. Verfahren nach Anspruch 1, wobei Schritt ii) weiterhin eine Probenverarbeitung unter Verwendung einer Offline-Festphasenextraktion (SPE), gefolgt von einer Online-SPE im 96-Well-Format aufweist.

11. Verfahren nach Anspruch 1, wobei das markierte IN-105 verwendet wird, um exogen bereitgestelltes IN-105 von endogenem Insulin zu unterscheiden.

12. Verfahren nach einem der vorhergehenden Ansprüche, das angewendet wird, um IN-105 über einen Konzentrationsbereich von 0,200 ng/ml bis 50,0 ng/ml zu bestimmen.

13. Verfahren nach Anspruch 1, aufweisend:
i) eine bekannte Menge von IN-105 durch ein stabiles Isotop markieren, um das markierte IN-105 zu bilden,
ii) das markierte IN-105 in die biologische Matrix einführen, wobei die biologische Matrix mindestens ein zusätzliches Insulin oder Insulinanalogon aufweist,
iii) die aus Schritt ii) erhaltene Lösung einem Mischphasen-Anionenaustausch-Umkehrphasen-Festphasenextraktionsverfahren unter Verwendung einer Offline-Festphasenextraktion (SPE) unterziehen, gefolgt von einer Online-SPE im 96-Well-Format, und
iv) das intakte markierte IN-105 relativ zu dem des mindestens einen zusätzlichen Insulintyps oder Insulinanalogons durch Flüssigphasenchromatographie-Tandem-Massenspektrometrie (LC-MS / MS) analysieren.

14. Verwendung von IN-105, markiert mit einem stabilen Stickstoffisotop, zur Bestimmung der Stabilität von IN-105 in einer biologischen Matrix, wobei die biologische Matrix mindestens einen zusätzlichen Insulintyp oder ein Insulinanalogon aufweist, wobei die Verwendung aufweist:
i) eine bekannte Menge von IN-105 durch ein stabiles Isotop markieren, um ein markiertes IN-105 zu bilden,
ii) das markierte IN-105 in die biologische Matrix einführen, und
iii) die aus Schritt ii) erhaltene Lösung einem Mischphasen-Anionenaustausch-Umkehrphasen-Festphasenextraktionsverfahren unter Verwendung einer Offline-Festphasenextraktion unterziehen, gefolgt von einer Online-Festphasenextraktion im 96-Well-Format, und
iv) das intakte markierte IN-105 relativ zu dem des mindestens einen zusätzlichen Insulintyps oder Insulinanalogons durch LC-MS/MS analysieren.

15. Verwendung nach Anspruch 14, wobei das stabile Isotop von Stickstoff ¹⁵N ist.

## Revendications

1. Procédé pour détecter et évaluer quantitativement de manière sélective de l'IN-105 dans une matrice biologique en présence de n'importe quelle insuline humaine ou insuline co-administrée, incluant de l'insuline glargine, dans lequel la matrice biologique est du sang total, du sérum sanguin, du plasma sanguin, de l'urine, du bouillon ou tampon de fermentation, dans lequel le procédé comprend les étapes de :
i. étiquetage d'une quantité connue d'IN-105 par un isotope stable pour former un IN-105 étiqueté ;
ii. introduction de l'IN-105 étiqueté dans la matrice biologique ; et
iii. analyse de la matrice biologique en ce qui concerne de l'IN-105 intact par chromatographie en phase liquide avec spectrométrie de masse en tandem (LC-MS/MS), dans laquelle l'IN-105 a la structure suivante :

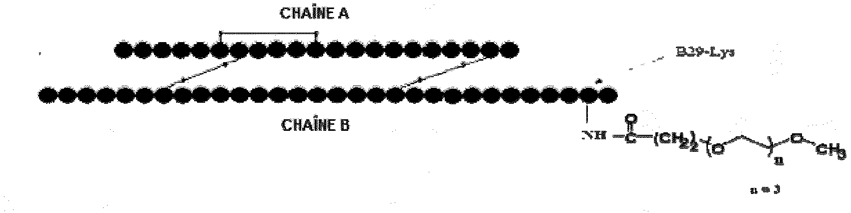

2. Procédé selon la revendication 1, dans lequel l'étiquetage d'une quantité connue d'IN-105 par un isotope stable pour former de l'IN-105 étiqueté comprend l'utilisation d'un milieu de culture comprenant une souche recombinée de *Pichia pastoris* portant un gène qui code pour l'expression d'IN-105 pendant un processus de fermentation.

3. Procédé selon la revendication 1 ou 2, dans lequel l'isotope est au moins l'un parmi de l'azote (¹⁵N), du soufre (³⁴S), de l'oxygène (¹⁸O), de l'hydrogène (²H) et du carbone (¹³C).

4. Procédé selon la revendication 3, dans lequel la source d'azote isotopique (¹⁵N) dans le milieu de culture est au moins l'une de sulfate d'ammonium, de phosphate d'ammonium, d'hydroxyde d'ammonium, de méthylamine, d'urée, ou dans lequel la source de carbone isotopique (¹³C) dans le milieu de culture est au moins l'une de méthanol, de glycérol, de glucose, de sorbitol, ou dans lequel la source de soufre isotopique (³⁴S) dans le milieu de culture est au moins l'une de sulfate de calcium, de sulfate de magnésium, de sulfate de potassium.

5. Procédé selon la revendication 2, dans lequel le processus de fermentation est un processus à deux phases, dans lequel la première phase est une phase discontinue pour augmenter la masse cellulaire avec l'inclusion de glycérol en tant que source de carbone et une phase discontinue alimentée utilisant du méthanol pour induire la sécrétion d'un précurseur de l'IN-105 étiqueté et la source d'isotope pour inclusion dans l'IN-105 étiqueté exprimé.

6. Procédé selon la revendication 5, dans lequel le précurseur d'IN-105 étiqueté est traité avec de la trypsine et de la carboxypeptidase B pour former l'IN-105 étiqueté.

7. Procédé selon la revendication 1, dans lequel la matrice biologique est combinée avec du di-potassium ou du tri-potassium EDTA.

8. Procédé selon la revendication 1, dans lequel la matrice biologique est stockée à -20 °C ou à -80 °C ou fraîchement préparée pour chaque cycle.

9. Procédé selon la revendication 10, dans lequel la période de stockage est de 1 jour à 250 jours.

10. Procédé selon la revendication 1, dans lequel l'étape ii) comprend en outre un traitement d'échantillon utilisant une extraction en phase solide (SPE) hors ligne suivie par une SPE en ligne dans un format à 96 puits.

11. Procédé selon la revendication 1, dans lequel l'IN-105 étiqueté est utilisé pour faire la distinction entre de l'IN-105 délivré de manière exogène et de l'insuline endogène.

12. Procédé selon l'une quelconque des revendications précédentes, utilisé pour déterminer de l'IN-105 sur une plage de concentrations de 0,200 ng/ml à 50,0 ng/ml.

13. Procédé selon la revendication 1, comprenant :
i) l'étiquetage d'une quantité connue d'IN-105 par l'isotope stable d'azote pour former l'IN-105 étiqueté ;
ii) l'introduction de l'IN-105 étiqueté dans la matrice biologique, dans laquelle la matrice biologique comprend au moins une insuline ou analogue d'insuline supplémentaire ;
iii) la soumission de la solution obtenue dans l'étape ii) à un processus d'extraction en phase solide en phase inverse par échange d'anions en mode mixte utilisant une extraction en phase solide (SPE) hors ligne suivie par une SPE en ligne dans un format à 96 puits ; et
iv) l'analyse de l'IN-105 étiqueté intact par rapport à celui de l'au moins un type supplémentaire d'insuline ou d'analogue d'insuline par chromatographie en phase liquide avec spectrométrie de masse en tandem (LC-MS/MS).

14. Utilisation d'IN-105, étiqueté avec un isotope stable d'azote, pour déterminer la stabilité d'IN-105 dans une matrice biologique, dans laquelle la matrice biologique comprend au moins un type supplémentaire d'insuline ou un analogue d'insuline, l'utilisation comprenant :
i) l'étiquetage d'une quantité connue d'IN-105 par un isotope stable d'azote pour former un IN-105 étiqueté,
ii) l'introduction de l'IN-105 étiqueté dans la matrice biologique,
iii) la soumission de la solution obtenue dans l'étape ii) à un processus d'extraction en phase solide en phase inverse à échange d'anions en mode mixte utilisant une extraction en phase solide hors ligne suivie par une extraction en phase solide en ligne dans un format à 96 puits, et
iv) l'analyse de l'IN-105 étiqueté intact par rapport à celui de l'au moins un type supplémentaire d'insuline ou d'analogue d'insuline par LC-MS/MS.

15. Utilisation selon la revendication 14, dans lequel l'isotope stable d'azote est ¹⁵N.
